# EUROPEAN PATENT SPECIFICATION

(11) **EP 1 681 026 B1**
(45) Date of publication and mention of the grant of the patent: **30.05.2012**
(21) Application number: 06000708.5
(22) Date of filing: 13.01.2006
(51) Int. Cl.: A61B 18/12

(54) **Electrosurgical generator using a full bridge topology**
Elektrochirurgischer Generator mit einer Vollbrückenschaltung
Générateur électrochirurgical avec un convertisseur à pont integral

(30) Priority: 13.01.2005 US 643734 P; 10.01.2006 US 328766 P
(43) Date of publication of application: 19.07.2006
(73) Proprietor: Covidien AG, 8212 Neuhausen am Rheinfall (CH)
(72) Inventor: Behnke, Robert, Erie Boulder Colorado (US)
(74) Representative: HOFFMANN EITLE

(56) References cited:
- EP-A- 0 880 220
- DE-A1- 19 848 540
- US-A- 6 093 186
- US-A1- 2004 095 100
- US-A1- 2004 097 914
- US-A1- 2004 138 654
- US-B1- 6 582 427

## Description

### CROSS-REFERENCE TO RELATED APPLICATION

The present application claims priority to a U.S. Provisional Application Serial No. 60/643,734 entitled "CIRCUIT AND METHOD FOR CONTROLLING AN ELECTROSURGICAL GENERATOR USING A FULL BRIDGE TOPOLOGY" by Robert Behnke filed on January 13, 2005, the entire contents of which is hereby incorporated by reference herein.

### BACKGROUND

### 1. Technical Field

The present disclosure is directed to electrosurgical systems, and, in particular, to a circuit and method for controlling an electrosurgical generator using a full bridge topology to control the radio frequency (RF) energy output.

### 2. Description of the Related Art

An electrosurgical generator is used in surgical procedures to deliver electrical energy to the tissue of a patient. When an electrode is connected to the generator, the electrode can be used for cutting, coagulating or sealing the tissue of a patient with high frequency electrical energy. During normal operation, alternating electrical current from the generator flows between an active electrode and a return electrode by passing through the tissue and bodily fluids of a patient.

The electrical energy usually has its waveform shaped to enhance its ability to cut, coagulate or seal tissue. Different waveforms correspond to different modes of operation of the generator, and each mode gives the surgeon various operating advantages. Modes may include cut, coagulate, a blend thereof, desiccate, or spray. A surgeon can easily select and change the different modes of operation as the surgical procedure progresses.

In each mode of operation, it is important to regulate the electrosurgical power delivered to the patient to achieve the desired surgical effect. Applying more electrosurgical power than necessary results in tissue destruction and prolongs healing. Applying less than the desired amount of electrosurgical power inhibits the surgical procedure. Thus, it is desirable to control the output energy from the electrosurgical generator for the type of tissue being treated.

Different types of tissues will be encountered as the surgical procedure progresses and each unique tissue requires more or less power as a function of frequently changing tissue impedance. As different types of tissue and bodily fluids are encountered, the impedance changes and the response time of the electrosurgical control of output power must be rapid enough to seamlessly permit the surgeon to treat the tissue. Moreover, the same tissue type can be desiccated during electrosurgical treatment and thus its impedance will change dramatically in the space of a very brief time. The electrosurgical output power control has to respond to that impedance change as well.

Two conventional types of power regulation are used in commercial electrosurgical generators. The most common type controls the DC power supply of the generator by limiting the amount of power provided from the AC mains to which the generator is connected. A feedback control loop regulates output voltage by comparing a desired voltage with the output voltage supplied by the power supply. Another type of power regulation in commercial electrosurgical generators controls the gain of the highfrequency or radio frequency amplifier. A feedback control loop compares the output power supplied from the RF amplifier for adjustment to a desired power level. Generators that have feedback control are typically designed to hold a constant output voltage, and not to hold a constant output power.

U.S. Pat. Nos. 3,964,487; 3,980,085; 4,188,927 and 4,092,986 have circuitry to reduce the output current in accordance with increasing load impedance. In those patents, constant voltage output is maintained and the current is decreased with increasing load impedance.

U.S. Pat. No. 4,126,137 controls the power amplifier of the electrosurgical unit in accord with a non-linear compensation circuit applied to a feedback signal derived from a comparison of the power level reference signal and the mathematical product of two signals including sensed current and voltage in the unit.

U.S. Pat. No. 4,658,819 has an electrosurgical generator which has a microprocessor controller based means for decreasing the output power as a function of changes in tissue impedance.

U.S. Pat. No. 4,727,874 includes an electrosurgical generator with a high frequency pulse width modulated feedback power control wherein each cycle of the generator is regulated in power content by modulating the width of the driving energy pulses.

U.S. Pat. No. 3,601,126 has an electrosurgical generator having a feedback circuit that attempts to maintain the output current at a constant amplitude over a wide range of tissue impedances.

EP-A2-0 880 220 discloses a full-bridge converter.

US2004/0138654 discloses an electrosurgical generator using a full bridge topology, the generator being coupled in a phase-locked loop and with over current protection comprising a current sense transformer configured to measure primary current at the primary side of a radio frequency transformer, a comparator and a monostable.

### SUMMARY

The invention is defined in claim 1 below. The dependent claims are directed to optional features and preferred embodiments.

An electrosurgical generator that uses a full bridge topology as the RF output stage to control the output RF energy and a control method therefore are provided. Most electrosurgical generators have a closed loop control signal that is derived from the difference of what is measured and what is desired. This control signal is feed back to the DC converter, which varies the DC voltage that feeds into the RF stage. The closed loop of the full bridge topology of the present disclosure uses the control signal that is derived from the RF energy that is measured and what is desired, to drive the RF output stage. The full bridge topology can be either pulse width driven or phase shift driven, in either case, as the control signal changes so will the amount of time the full bridge will place voltage across the primary of the RF transformer. Varying the amount of time the voltage is imposed across the transformer's primary winding controls the amount of energy on the RF transformer. This voltage is a square wave whose pulse length varies in relation to the control signal. The corresponding energy on the secondary side of the RF transformer is filtered, usually at the operating frequency, to produce a sinusoidal waveform. Using a full bridge topology, the RF energy can be controlled directly at the RF output stage instead of using a varying DC input. This allows the DC input to be at a steady level or an unregulated level and, therefore, the DC converter can be removed from the closed loop of the generator.

The advantage of using a full bridge topology for the RF stage is that the DC voltage no longer controls the output RF energy. This means the DC converter can be removed from the electrosurgical generator, or the DC level can be preset such as the closed loop of the electrosurgical generator will not control it during operation. By removing the DC converter from the closed loop has the advantage of a much faster closed loop response of the electrosurgical generator. The other advantage of using the full bridge topology is the RF transformer is driven symmetrically which makes the output stable to all loads.

According to one embodiment of the present disclosure, a system for controlling an electrosurgical generator using a full bridge topology is disclosed. The system includes a high voltage direct current power source which supplies power and a radio frequency output stage which receives power from the high voltage direct current power source and outputs radio frequency energy at a predetermined radio frequency set point. The system also includes one or more sensors which determine one or more parameters of radio frequency energy being applied to tissue and a microprocessor configured to receive one or more parameters and outputs the predetermined radio frequency set point to the radio frequency output stage as a function of one or more of the parameters of radio frequency energy.

According to an example of the present disclosure, a method for controlling an electrosurgical generator using a full bridge topology is disclosed. The method includes the steps of supplying power from a high voltage direct current power source and receiving power from the high voltage direct current power source at a radio frequency output stage. The method also includes the steps of outputting radio frequency energy at a predetermined radio frequency set point and determining one or more parameters of radio frequency energy being applied to a load. The method further includes the step of receiving the one or more parameters at a microprocessor and outputting the predetermined radio frequency set point to the radio frequency output stage as a function of the one or more of the parameters.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1 is a block diagram of a radio frequency (RF) output control circuit of a conventional electrosurgical generator;
FIG. 2 is a block diagram of RF output control circuit of an electrosurgical generator according to the teachings of the present disclosure;
FIG. 3 is a schematic diagram of an RF output stage according to an embodiment of the present disclosure;
FIG. 4 is a schematic diagram of an over-current protection circuit;
FIG. 5 is a schematic diagram of a first over-voltage protection circuit;
FIG. 6 is a schematic diagram of a second over-voltage protection circuit;
FIG. 7 is a schematic diagram of a conventional RF output stage circuit;
FIG. 8 illustrates several views of how the RF output stage can be modeled over time; and
FIGS. 9A-B illustrate several views of output curves after experiencing arcing.

### DETAILED DESCRIPTION

Embodiments of the present disclosure will be described herein below with reference to the accompanying drawings. In the following description, well-known functions or constructions are not described in detail to avoid obscuring the invention in unnecessary detail. In the figures, like reference numerals represent like elements.

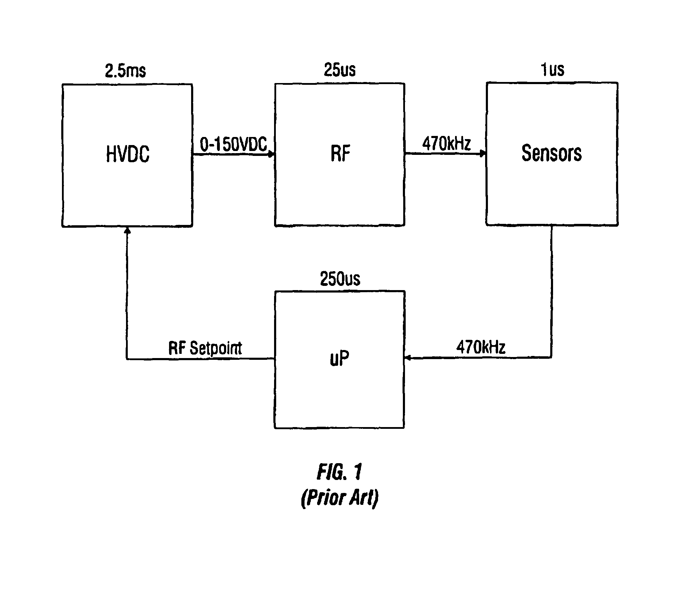

The speed of any electrosurgical generator is governed by the slowest block or component in the control loop. In conventional electrosurgical generator topology as shown in FIG. 1, the high voltage DC power source (HVDC) response time is 10 times slower than the intended microprocessor (uP) loop speed, e.g., 2.5ms vs. 250us. This results in the microprocessor waiting for the HVDC to respond thereby not utilizing faster response times offered by electronic circuitry.

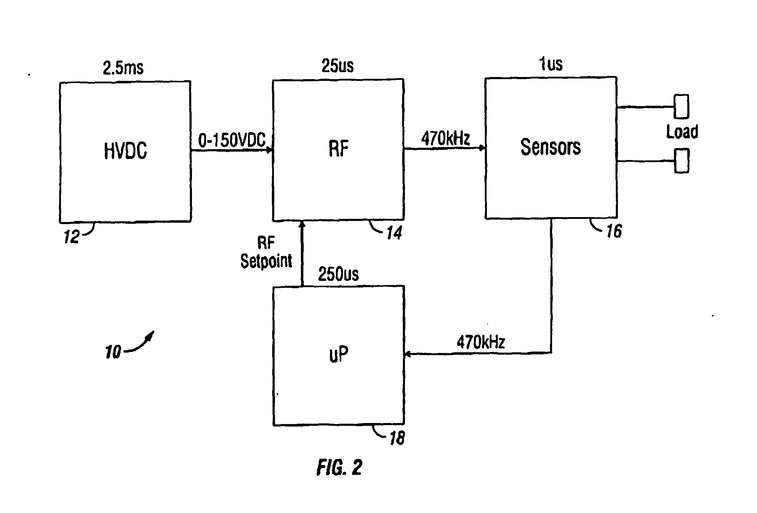

Referring to FIG. 2, an electrosurgical generator according to the present disclosure is illustrated. The electrosurgical generator 10 includes a high voltage DC power source (HVDC) 12 for supplying power to the generator, an RF output stage 14 for receiving power from the HVDC 12 and outputting RF power at a predetermined set point, sensors 16 for determining parameters, e.g., current and voltage, of power being applied to a load, e.g., tissue, and a microprocessor 18 for controlling the overall operations of the electrosurgical generator 10 and for receiving the measured current and voltage and outputting a set point to the RF output stage 14.

In embodiments, the HVDC 12 is set to a fixed voltage level. The response of the HVDC 12 becomes a concern only at initialization of the generator 10. The RF output stage's response time with a phase shift controlled topology is now 10 times faster than the microprocessor control loop speed, as will be described below. The limiting factor is no longer the HVDC, but the speed of the microprocessor control loop, which is 10 times faster than the intended microprocessor (uP) loop speed, e.g., 2.5ms vs. 250us. It is envisioned that the difference will be even larger as more advanced microprocessors are developed and implemented.

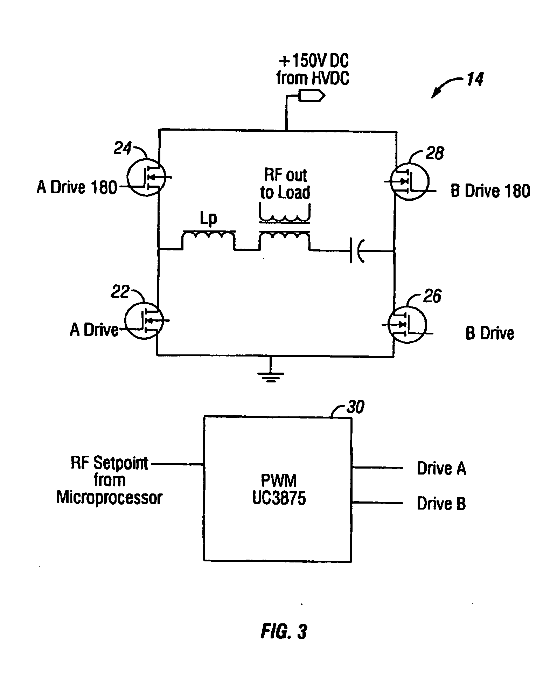

Referring to FIG. 3, the RF output stage is illustrated in more detail. The RF output stage 14 includes a transformer 20 and a plurality of transistors 22, 24, 26, 28 for adjusting the RF output power of the transformer 20 configured in a full bridge topology. The RF output stage 14 further includes a pulse-width modulator 30 for driving the plurality of transistors 22, 24, 26, 28. The pulse width modulator (PWM) 30 has at least two drive outputs, e.g., Drive A and Drive B. Drive A is coupled to transistor 22 and Drive A shifted 180 degrees is coupled to transistor 24. Similarly, Drive B is coupled to transistor 26 and Drive B shifted 180 degrees is coupled to transistor 28. A push-pull typology is used to accomplish voltage from DC to RF conversion. Two gate drive signals that are 180° out of phase are used to drive the plurality of transistors 22, 24, 26, 28, called T_ON and T_ON_180. The gate drive signals turn on each of the FET's at opposite times to deliver a waveform at the specified power. The pulse width modulator 30 includes an input for receiving the RF output set point from microprocessor 18. Alternatively, the transistors may be phase shift driven from a single control.

Since, the RF output stage 14 responds to a dynamic load ten times faster than the microprocessor, over-voltage and over-current conditions may occur at the load. Therefore, three hardware solutions have been implemented to help minimize these conditions.

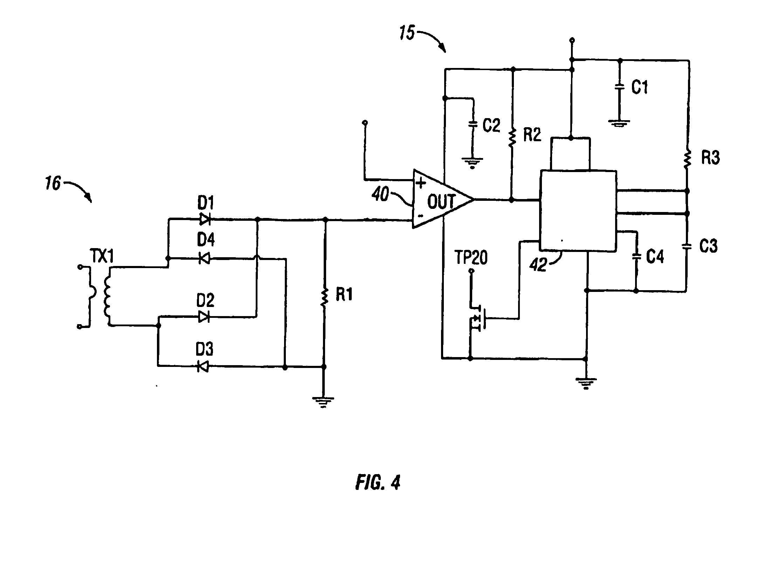

Over-current protection is implemented by an over-current protection circuit 15 shown in FIG. 4. Referring to FIG. 4, current sense transformer TX1 located on the sense board 16 measures the output current to the load. The sensed current is input to comparator 40 which is then compared to a reference voltage Vref. If a fault condition occurs, such as arcing, the comparator 40 will determine that the sensed current is greater than the reference voltage Vref and will trigger the RF set point to be pulled low through transistor M1 via connection point TP26. A timer 42, e.g., a 555 timer, will keep the RF set point at 0V for a predetermined period of time, e.g., 150uS, to effectively reduce the RF output from the RF output stage 14. Alternatively, the timer could be replaced by a flip flop that will pull down the RF set point by half and send an alarm to the microprocessor 18. Once the microprocessor 18 handles the alarm, the microprocessor 18 would reset the flip flop.

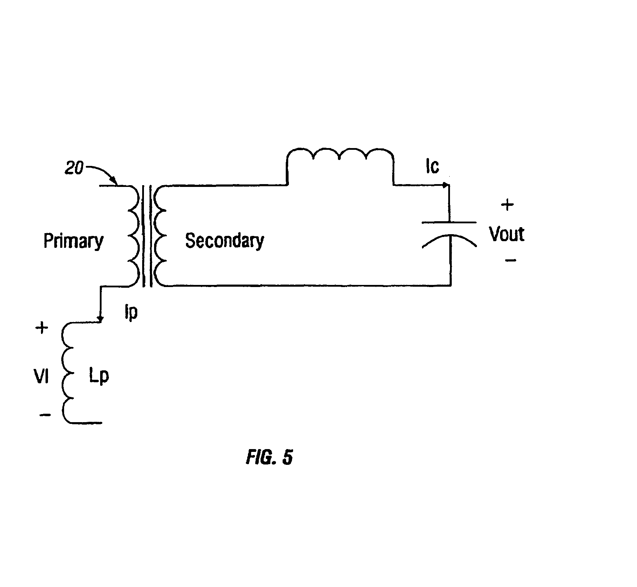

Over-voltage conditions may occur if low impedance is sensed, e.g., when a surgical instrument is removed from the operative site, and the generator attempts to increase the output voltage. Referring to FIG. 5, over-voltage protection is achieved by adding an inductor Lp to the primary side of the RF transformer 20. As the voltage on the output increases so will the current flowing through the output capacitor Vout. The primary current (Ip) equals the secondary current because it is dominated by the capacitor current (Ic). As Ip increases, the voltage that is across the primary inductor Lp will also increase. The voltage across the inductor will subtract from the voltage across the transformer which will reduce the amount of voltage to the secondary and as that is reduced, so is the amount of output voltage. This protection works cycle by cycle, will prevent a runaway condition of the output voltage and prevent a full-bore output from occurring at the load.

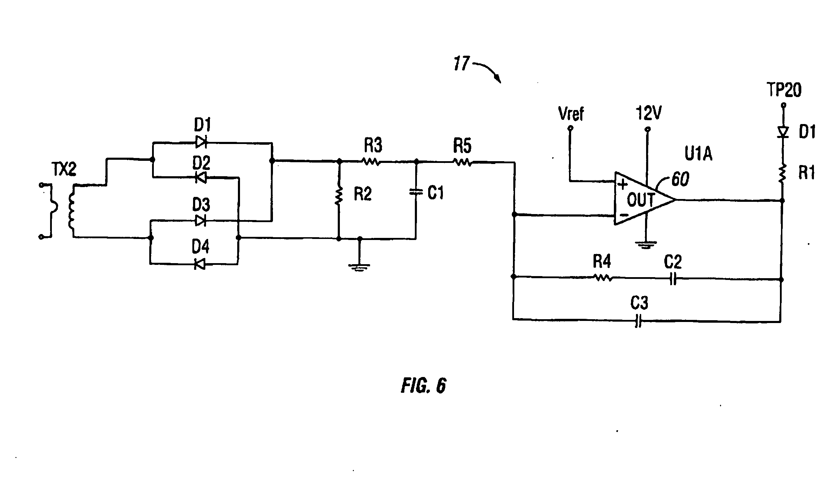

Additionally, over-voltage protection will be implemented to limit the amount of overshoot while an instrument goes from a low to high impedance. This over-voltage protection scheme is implemented in an over-voltage protection circuit 17 shown in FIG. 6. Transformer TX2 monitors the current on the primary winding of the RF transformer 20, or alternatively, will monitor the output voltage from the sense board. As described above, when the primary current increases so will the voltage at the output. Since the capacitance is a constant, the primary current can be calculated such that a certain voltage will be across the output capacitance. Once this level goes above a specific value, e.g., Vref, comparator 60 will start to pull down the RF set point through diode D1 via connection point TP26. This protection has the capability of changing the level of overshoot by changing the value of variable resistor R2, which can be switched in. The speed of this protection is about 10Khz, or 100us. This is limited to about 10% over the voltage limit which will allow the microprocessor to see the error and adjust the set point accordingly.

The generator employing a full bridge topology of the present disclosure has several advantages over conventional generators which control the RF output by varying the high voltage power supply (HVDC). For example, the stored energy on the HVDC does not effect the output of the generator. Currently, the generator output is left on for a few milliseconds after the end of an activation to help bleed off the voltage to prevent excess energy storage in the generator. The higher the impedance present at the output, the longer it takes to dissipate the voltage on the HVDC. The danger here is that the RF board may get turned off but the HVDC may still have stored energy, causing inadvertent errors to be reported. Additionally, as RF power requirements increase so will the capacitor off of the HVDC. Currently, this stored voltage in the capacitor needs to be discharged after each activation. Because the set point turns the RF off at the PWM instead of the HVDC, residual voltage on the HVDC isn't an issue at the end of an activation.

Furthermore, an RF amplifier of the electrosurgical generator will have no tuned elements. The advantage of having no tuned elements on the RF amplifier is that the operating frequency of the output stage module will be completely independent of the operating frequency of the RF amplifier. Frequency, instead, becomes dependent on the resonance of each of the separate output modes of the generator, e.g., coagulate, cut, blend, etc. The advantage of this configuration is that the output frequency can be changed without re-tuning the RF output stage only re-tuning the output module relating to a particular mode, facilitating any modifications or troubleshooting. This delegation of resonating components places much less stress on the FETs during arcing and the RF output stage will be able to force the output to be more stable as will be described below.

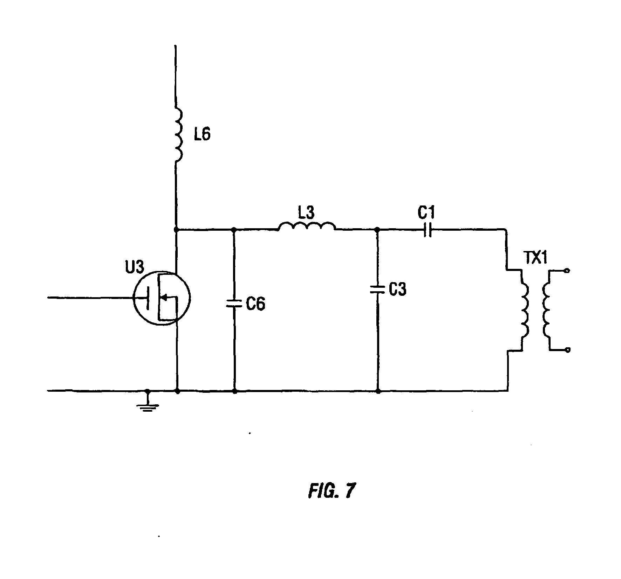

The current topology is a class E, where the FET resonates with the tank, capacitor C6, capacitor C3, and inductor L3, as shown in FIG. 7. When an arcing condition hits the tank, this can short out the capacitor C3 thereby causing the resonance of this system to change dramatically. Any stored energy in inductor L6, the RF choke, will be released causing the voltage across the FET to increase. This voltage will eventually be clamped by the avalanche mode of the FET, for example, in conventional topology this is around 570V for a 500V rated FET. In the topology of the present disclosure, since there is no tank or RF choke, the voltage will not increase. Because of this, the generator is able to use 200V rated FETs. The lower voltage rated FETs have an advantage with lower Rds on and gate charge, which have less dissipatation and are less expensive than higher voltage rated FETs,

Additionally, the current conventional class E topology is efficient only in a narrow range of load impedances. As the load changes so will the waveform across the FET. This will cause symmetry issues of the waveform at the output. The topology of the present disclosure has an output filter which is optimized for certain voltages and currents. Because the FETs themselves are not tuned, the RF amplifier to the filter is unaffected by load changes.

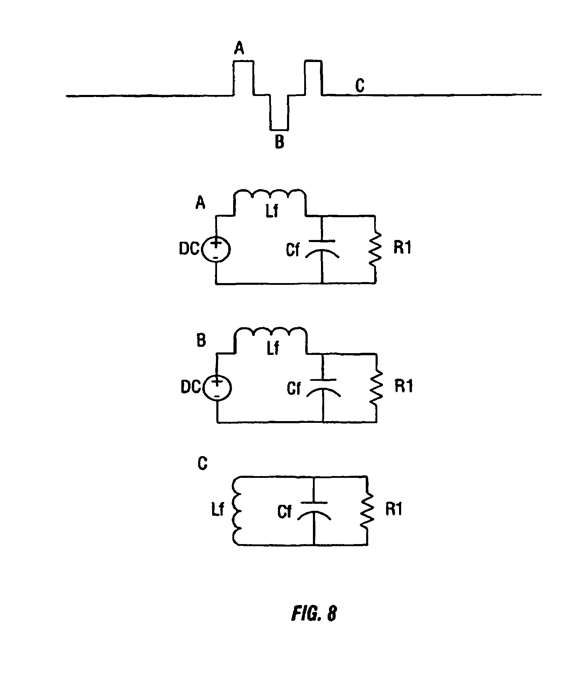

FIG. 8 illustrates how the transformer voltage, or output of the RF, is induced on to the output filter. During time A, the voltage positively charges the inductor Lf and capacitor Cf. During time B, the inductor Lf and capacitor Cf are of the opposite polarity. Time C shows that when there is no output voltage, the inductor Lf is in parallel with the capacitor Cf which will keep the same resonant frequency. If the load is shorted, the max current = (V*t)/L.

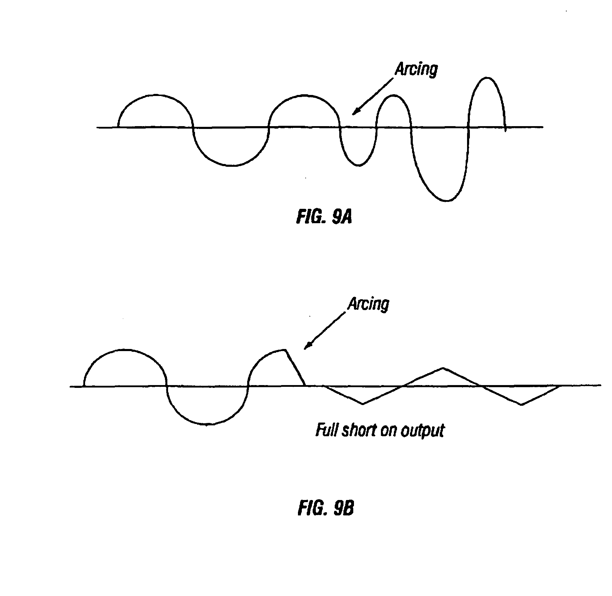

As mentioned above, an arcing condition in the existing class E topology causes the resonant frequency to change, subsequently changing the output frequency as illustrated in FIG. 9A. Because the topology of the present disclosure is using a transformer that is symmetrical on the primary side, it forces the secondary to be symmetrical even during arcing, as shown in FIG. 9B. When the output capacitor is shorted on the new topology, the series inductance acts as a resistor and it limits the current to the output.

Furthermore, the generator of the present disclosure reduces leakage current. Leakage current is dependant on several variables. The component that contributes most to leakage current is capacitor coupling from the primary to secondary winding of the RF transformer. As the voltage increases across the transformer, the capacitance will start to conduct current. The advantage of the full bridge RF topology is that it employs a 1-to-1 transformer and only has a maximum voltage of 150V, compared to 550 VRMS or 778 Vpeak of the transformers used in conventional generators. Because of the reduced voltage, the number of turns is reduced to about 5 turns thereby reducing the coupling capacitance.

While several embodiments of the disclosure have been shown in the drawings, it is not intended that the disclosure be limited thereto, as it is intended that the disclosures be as broad in scope as the art will allow and that the specification be read likewise. Therefore, the above description should not be construed as limiting, but merely as exemplifications of preferred embodiments.

## Claims

1. An electrosurgical generator (10) comprising a system using a full bridge topology, the system comprising:
a high voltage direct current power source (12) which supplies power;
a radio frequency output stage (14) which receives power from the high voltage direct current power source and outputs radio frequency energy at a predetermined radio frequency set point;
at least one sensor (16) which determines at least one parameter of the radio frequency energy being applied to a load; and
a microprocessor (18) configured to receive the at least one parameter of the radio frequency energy and output the predetermined radio frequency set point to the radio frequency output stage as a function of the at least one parameter of the radio frequency energy;
wherein the radio frequency output stage further comprises:
a radio frequency transformer (20) having a primary and a secondary side;
an output capacitor (Vout) operatively coupled in parallel to the radio frequency output stage at the secondary side of the radio frequency transformer, the output capacitor being configured to maintain equality between primary current at the primary side and secondary current at the secondary side;
an inductor (Lp) operatively coupled to the primary side of the radio frequency transformer configured to subtract primary voltage at the primary side from voltage crossing the transformer between the primary and secondary thereby reducing output voltage; and
an over-voltage protection circuit (17) including a second current sense transformer (TX2) configured to measure primary current at the primary side of the radio frequency transformer and a second comparator (60) configured to compare the primary current to a reference voltage, wherein if the output current is greater than the reference voltage, the microprocessor being configured to adjust the predetermined radio frequency set point.

2. A system according to claim 1, wherein the high voltage direct current power source is set to supply power at a fixed voltage level.

3. A system according to claim 1, wherein radio frequency output stage includes:
a plurality of transistors (22, 4, 6, 8) configured to adjust the radio frequency energy of a transformer (20); and
a pulse-width modulator (30) configured to drive the plurality of transistors, wherein the pulse width modulator includes at least a first drive output (A, B) and a second drive output (A, B), the pulse width modulator further comprising an input for receiving the predetermined radio frequency set point from the microprocessor.

4. A system as in claim 1, wherein the predetermined radio frequency set point is independent of the fixed voltage level.

5. A system as in claim 1, wherein the predetermined radio frequency set point is from about 0.7V to about 3.7V.

6. A system as in claim 1, further comprising:
an over-current protection circuit (15) including a first current sense transformer (TX1) which measures output current to the load and a first comparator (40) which compares the output current to a reference voltage, wherein if the output current is greater than the reference voltage the microprocessor sets the predetermined radio frequency set point to 0V for a predetermined period of time.

## Patentansprüche

1. Elektrochirurgischer Generator (10) mit einem System, das eine Vollbrückenschaltung verwendet, wobei das System aufweist:
eine Hochspannungsgleichstromquelle (12), die Strom zuführt;
eine Hochfrequenz-Ausgangsstufe (14), die Leistung von der Hochspannungsgleichstromquelle empfängt und Hochfrequenzenergie bei einem festgelegten Hochfrequenzsollwert ausgibt;
mindestens einen Sensor (16), der mindestens einen Parameter der auf eine Last angewandten Hochfrequenzenergie bestimmt; und
einen Mikroprozessor (18), der eingerichtet ist, um dem mindestens einen Parameter der Hochfrequenzenergie zu empfangen und den festgelegten Hochfrequenz-Sollwert an die Hochfrequenz-Ausgangsstufe als Funktion des mindestens einen Parameters der Hochfrequenzenergie auszugeben;
wobei die Hochfrequenz-Ausgangsstufe des Weiteren aufweist:
einen Hochfrequenzwandler (20) mit einer primären und einer sekundären Seite;
einen Ausgangskondensator (Vout), der mit der Hochfrequenz-Ausgangsstufe an der sekundären Seite des Hochfrequenzwandlers parallel wirkend gekoppelt ist, wobei der Ausgangskondensator eingerichtet ist, um ein Gleichgewicht zwischen einem primären Strom an der primären Seite und einem sekundären Strom an der sekundären Seite aufrecht zu erhalten;
einen Induktor (Lp), der mit der primären Seite des Hochfrequenzwandlers zusammenwirkend gekoppelt ist, die eingerichtet ist, eine primäre Spannung an der primären Seite von einer Spannung, die den Wandler zwischen der primären und sekundären durchquert zu subtrahieren und **dadurch** die Ausgangsspannung zu reduzieren; und
einen Überspannungsschutzschaltkreis (17) mit einem zweiten Current-Sense-Transformer (TX2), der eingerichtet ist, einen primären Strom an der primären Seite des Hochfrequenzwandlers zu messen, und einem zweiten Komparator (60), der eingerichtet ist, den primären Strom mit einer Referenzspannung zu vergleichen, wobei der Mikroprozessor eingerichtet ist, um den festgelegten Hochfrequenz-Sollwert einzustellen, wenn der Ausgangsstrom größer als die Referenzspannung ist.

2. System gemäß Anspruch 1, bei dem die Hochspannungsgleichstromquelle eingestellt ist, Leistung mit einem festgelegten Spannungspegel zuzuführen.

3. System gemäß Anspruch 1, bei dem die Hochfrequenz-Ausgangsstufe aufweist:
eine Vielzahl von Transistoren (22, 4, 6, 8), die eingerichtet sind, die Hochfrequenzenergie eines Wandlers (20) einzustellen; und
einen Pulsweitenmodulator (30), der eingerichtet ist, die Vielzahl von Transistoren zu steuern, wobei der Pulsweitenmodulator mindestens einen ersten Steuerungsausgang (A, B) und einen zweiten Steuerungsausgang (A, B) aufweist und der Pulsweitenmodulator des Weiteren einen Eingang zum Empfangen des festgelegten Hochfrequenz-Sollwerts von dem Mikroprozessor aufweist.

4. System gemäß Anspruch 1, bei dem der festgelegte Hochfrequenz-Sollwert unabhängig von dem festgelegten Spannungspegel ist.

5. System gemäß Anspruch 1, bei dem der festgelegte Hochfrequenz-Sollwert von in etwa 0,7 V bis in etwa 3,7 V ist.

6. System gemäß Anspruch 1, des Weiteren mit:
einem Überspannungsschutzschaltkreis (15), der einen ersten Current-Sense-Transformer (TX1), der einen Ausgangsstrom zu der Last misst, und einen ersten Komparator (40), der den Ausgangsstrom mit einer Referenzspannung vergleicht, aufweist, wobei der Mikroprozessor den festgelegten Hochfrequenz-Sollwert für eine festgelegte Zeitspanne auf 0V einstellt, wenn der Ausgangsstrom größer als die Referenzspannung ist.

## Revendications

1. Générateur électrochirurgical (10) comprenant un système utilisant une topologie pont complet, le système comprenant:
une source de puissance de courant continu de haute tension (12) qui fournit de la puissance;
un étage de sortie de radiofréquence (14) qui reçoit la puissance de la source de puissance de courant direct de haute tension et émet de l'énergie radiofréquence à un point réglé de radiofréquence prédéterminé;
au moins un capteur (16) qui détermine au moins un paramètre de l'énergie radiofréquence appliquée à une charge; et
un microprocesseur (18) configuré pour recevoir le au moins un paramètre de l'énergie radiofréquence et pour émettre le point réglé de radiofréquence prédéterminé à l'étage de sortie de radiofréquence en tant que fonction du au moins un paramètre de l'énergie radiofréquence;
où l'étage de sortie de radiofréquence comprend en outre:
un transformateur de radiofréquence (20) ayant un côté primaire et un côté secondaire;
un condensateur de sortie (Vout) fonctionnellement couplé en parallèle à l'étage de sortie de radiofréquence au côté secondaire du transformateur de radiofréquence, le condensateur de sortie étant configuré pour maintenir une égalité entre le courant primaire au côté primaire et le courant secondaire au côté secondaire;
une inductance Lp fonctionnellement couplée au côté primaire du transformateur de radiofréquence configurée pour déduire la tension primaire au côté primaire d'une tension croisant le transformateur entre les côtés primaire et secondaire en réduisant ainsi la tension de sortie; et
un circuit de protection contre la surtension (17) incluant un deuxième transformateur de sens de courant (TX2) configuré pour mesurer le courant primaire au côté primaire du transformateur de radiofréquence et un deuxième comparateur (60) configuré pour comparer le courant primaire à une tension de référence, où, si le courant de sortie est supérieur à la tension de référence, le microprocesseur étant configuré pour ajuster le point réglé de radiofréquence prédéterminé.

2. Système selon la revendication 1, dans lequel la source de puissance de courant continu de haute tension est réglée pour fournir de la puissance à un niveau de tension fixe.

3. Système selon la revendication 1, dans lequel l'étage de sortie de radiofréquence comprend:
une pluralité de transistors (22, 4, 6, 8) configurés pour ajuster l'énergie de radiofréquence d'un transformateur (20); et
un modulateur de largeur d'impulsion (30) configuré pour entraîner la pluralité de transistors, où le modulateur de largeur d'impulsion comprend au moins une première sortie d'entraînement (A, B) et une deuxième sortie d'entraînement (A, B), le modulateur de largeur d'impulsion comprenant en outre une entrée pour recevoir le point réglé de radiofréquence prédéterminé du microprocesseur.

4. Système selon la revendication 1, dans lequel le point réglé de radiofréquence prédéterminé est indépendant du niveau de tension fixé.

5. Système selon la revendication 1, dans lequel le point réglé de radiofréquence prédéterminé est d'environ 0,7 V à environ 3,7 V.

6. Système selon la revendication 1, comprenant en outre:
un circuit de protection contre une surintensité (15) incluant un premier transformateur de sens de courant (TX1) qui mesure le courant de sortie à la charge, et un premier
comparateur (40) qui compare le courant de sortie avec une tension de référence, où, si le courant de sortie est supérieur à la tension de référence, le microprocesseur établit le point réglé de radiofréquence prédéterminé à 0V pendant une période de temps prédéterminée.
